# EUROPEAN PATENT APPLICATION

(11) **EP 3 933 048 A1**
(43) Date of publication of application: **05.01.2022**
(21) Application number: 20179108.4
(22) Date of filing: 09.06.2020
(51) Int. Cl.: C12Q 1/6844

(54) **METHOD FOR DETERMINING PRESENCE OF A PRE-DETERMINED NUCLEIC ACID SEQUENCE IN ANIMAL SAMPLES**

(71) Applicant: LiVET AG, 3018 Bern (CH)
(72) Inventor: Zürcher, Samuel, 3018 Bern (CH); Lüthi, Alexander, 3018 Bern (CH); Weibel, Lea, 3018 Bern (CH)
(74) Representative: Vossius & Partner Patentanwälte Rechtsanwälte mbB

(57) **Abstract**

The present invention relates to a method for determining presence of a pre-determined nucleic acid sequence in a sample, the method comprising the steps of adding one or more enzyme(s) providing activities of RNA- and/or DNA-dependent DNA polymerase activity and strand-displacement activity to the sample to be analysed for the presence of the pre-determined nucleic acid sequence; adding at least five DNA primers to the sample to be analysed for the presence of the pre-determined nucleic acid sequence, wherein at least one DNA primer comprises a sequence hybridisable to the nucleic acid sequence and at least one DNA primer comprises a sequence hybridisable to the DNA sequence reverse-complementary to the nucleic acid sequence; incubating the sample resulting at a fixed temperature; determining whether an elongated DNA sequence is present in the sample, wherein presence of the elongated DNA sequence in the sample is indicative of the presence of the pre-determined nucleic acid sequence in the sample, wherein the sample is obtained from an animal.

## Description

The present invention relates to a method for determining presence of a pre-determined nucleic acid sequence in a sample, the method comprising the steps of adding one or more enzyme(s) providing activities of RNA- and/or DNA-dependent DNA polymerase activity and strand-displacement activity to the sample to be analysed for the presence of the pre-determined nucleic acid sequence; adding at least five DNA primers to the sample to be analysed for the presence of the pre-determined nucleic acid sequence, wherein at least one DNA primer comprises a sequence hybridisable to the nucleic acid sequence and at least one DNA primer comprises a sequence hybridisable to the DNA sequence reverse-complementary to the nucleic acid sequence; incubating the sample resulting at a fixed temperature; determining whether an elongated DNA sequence is present in the sample, wherein presence of the elongated DNA sequence in the sample is indicative of the presence of the pre-determined nucleic acid sequence in the sample, wherein the sample is obtained from an animal.

Various methods exist for determining whether nucleic acid sequences are present in samples obtained from animals. Most methods rely on nucleic acid molecules hybridisable to a pre-determined sequence, such as for example a sequence expected to be present in a pathogen.

However, existing tests are either highly sensitive and specific but slow or quickly provide a result which lacks appropriate reliability. Thus, there is a need in the art for a method providing a highly sensitive and specific and quick result.

The above technical problem is solved by the embodiments provided herein and as characterized in the claims.

Accordingly, the present invention relates to, inter alia, the following embodiments.
1. A method for determining presence of a pre-determined nucleic acid sequence in a sample, the method comprising the steps of:
   (a) adding one or more enzyme(s) providing activities of RNA- and/or DNA-dependent DNA polymerase activity and strand-displacement activity to the sample to be analysed for the presence of the pre-determined nucleic acid sequence;
   (b) adding at least five DNA primers to the sample to be analysed for the presence of the pre-determined nucleic acid sequence, wherein at least one DNA primer comprises a sequence hybridisable to the nucleic acid sequence and at least one DNA primer comprises a sequence hybridisable to the DNA sequence reverse-complementary to the nucleic acid sequence;
   (c) incubating the sample resulting from steps (a) and (b) at a fixed temperature;
   (d) determining whether an elongated DNA sequence is present in the sample, wherein presence of the elongated DNA sequence in the sample is indicative of the presence of the pre-determined nucleic acid sequence in the sample
   wherein the sample is obtained from an animal.
2. The method of embodiment 1, wherein four of the at least five primers are forward inner primer (FIP), backward inner primer (BIP), loop primer forward (LPF) and loop primer backwards (LPB), respectively.
3. The method of embodiment 1 and 2, wherein the fifth primer is a B3 primer.
4. The method of any one of embodiments 1 to 3, wherein no F3 primer is used.
5. The method of any one of embodiments 1 to 4, wherein the pre-determined nucleic acid sequence is an RNA or DNA sequence.
6. The method of any one of embodiments 1 to 5, wherein the pre-determined RNA or DNA sequence is comprised in a pathogen.
7. The method of embodiment 6, wherein the pathogen is a virus, a bacterium, a fungus or a parasite.
8. The method of any one of embodiments 1 to 7, wherein the fixed temperature is between 50 and 75°C.
9. The method of any one of embodiments 1 to 8, wherein the sample in step (c) is incubated for 1 to 120 minutes.
10. The method of any one of embodiments 1 to 9, wherein presence of the double-stranded elongated DNA sequence in the sample is determined by using a nucleic acid molecule hybridisable to the double-stranded elongated DNA sequence, in particular wherein the nucleic acid molecule is labelled, using a molecule that intercalates in the double-stranded elongated DNA sequence or using turbidity measurement.
11. A method of treating an animal infected by a pathogen, the method comprising administering to the subject an efficient amount of a therapeutic drug, wherein the subject has previously been determined to be infected by the pathogen using the method of any one of embodiments 1 to 10.
12. The method of embodiment 11 wherein the animal is a horse.
13. The method of embodiment 11 or 12, wherein the pathogen is a virus, a bacterium, a fungus or a parasite.
14. The method of any one of embodiments 11 or 13, wherein the therapeutic drug is an antiviral, antibiotic, antifungal or antiparasitic drug, respectively.
15. The method of any one of embodiments 11 to 14, wherein the pathogen is *Streptococcus equi equi, Equine herpes virus,* in particular *Equine herpes virus 1* or *4*, or *Equine influenza virus.*

Accordingly, in a first aspect, the invention relates to a method for determining presence of a pre-determined nucleic acid sequence in a sample, the method comprising the steps of adding one or more enzyme(s) providing activities of RNA- and/or DNA-dependent DNA polymerase activity and strand-displacement activity to the sample to be analysed for the presence of the pre-determined nucleic acid sequence; adding at least five DNA primers to the sample to be analysed for the presence of the pre-determined nucleic acid sequence, wherein at least one DNA primer comprises a sequence hybridisable to the nucleic acid sequence and at least one DNA primer comprises a sequence hybridisable to the DNA sequence reverse-complementary to the nucleic acid sequence; incubating the sample resulting at a fixed temperature; determining whether a double-stranded elongated DNA sequence is present in the sample, wherein presence of the double-stranded elongated DNA sequence in the sample is indicative of the presence of the pre-determined nucleic acid sequence in the sample, wherein the sample is obtained from an animal.

It is preferred within the methods of the present invention that five DNA primers are used.

Within the present invention, it is preferred that the at least five primers comprise a forward inner primer (FIP), backward inner primer (BIP), loop primer forward (LPF) and loop primer backwards (LPB), respectively. Preferably, the at least five primers further comprise a B3 primer. In such an embodiment, it is preferred that no F3 primer is used. These terms are commonly used in methods related to loop-mediated isothermal amplification (LAMP) methods, such as those described by Nagamine et al. 2002. Molecular and Cellular Probes 16. 223-229

Within the present invention, it was surprisingly found that a five-primer system is most efficient in detecting a pre-determined nucleic acid sequence. "Most efficient" as used herein means that detection is as fast and sensitive as commonly used techniques but maintains reliability, which is a prerequisite in tests used for detecting nucleic acids such as nucleic acids derived from animal samples. In addition, it was found that by using five primers instead of six primers as in the standard LAMP technology, shorter target sequences can be detected.

Within the methods of the present invention, it is thus preferred that the fifth primer is a B3 primer. It is also preferred that no F3 primer is used. This is because it was surprisingly found by the inventors that in the presence of a B3 primer but absence of an F3 primer, detection is as fast and as sensitive. Using the methods of the present invention, detection was observed to be possible within ten minutes and as sensitive to detect a low number of pre-determined nucleic acid sequence in a sample. That is, as it is shown in the appended Examples, a positive detection of a pre-determined nucleic acid sequence was achieved using five primers, in particular FIP, BIP, LPF, LPB and B3, within ten minutes after addition of primers and enzymes. Accordingly, the methods of the present invention, for the first time, provide a reliable and fast way to detect infections which is important in controlling outbreak of the same.

The use of less than 6 primers is preferred within the methods of the present invention. The use of less primers requires less primer binding sites and thus shorter target sequences can be detected. This is particularly important since the availability of target sequence, in particular conserved and target specific sequences, is highly limited. In this respect, it was now surprisingly found that the specific methods as provided herein provide the same sensitivity when using 5 primers instead of 6 primers. This surprising demonstration has the further advantage in cases where more than one primer system is used in the same method, e.g. for detecting more than one target sequence in the same or different pathogen.

Within the methods of the present invention, one or more enzyme(s) providing activities of RNA- and/or DNA-dependent DNA polymerase activity and strand-displacement activity are used. That is, in case of an RNA sequence, all three activities are to be added to the RNA sequence to be analyzed. In case of a DNA sequence, activity of the RNA-dependent DNA polymerase is not required. The activities can be provided by one enzyme having all two/three activities, or several enzymes each having one or more of the two/three activities.

It is preferred that the pre-determined nucleic acid sequence is an RNA or DNA sequence.

In some embodiments, it is preferred that the pre-determined RNA or DNA sequence is comprised in a pathogen. That is, the method provided herein is used to detect presence of a nucleic acid sequence of a pathogen in a sample obtained from an animal. The invention thus relates to, inter alia, a method for diagnosing whether an animal suffers or is likely to suffer from a disease caused by a pathogen, wherein presence of a nucleic acid sequence of said pathogen was determined using the methods provided herein.

It is preferred that the animal is a mammal, preferably a horse. The animal is not a human.

In some embodiments, the pathogen is a virus, a bacterium, a fungus or a parasite. It is particular preferred that the pathogen is *Streptococcus equi equi, Equine herpes virus,* in particular *Equine herpes virus 1* or *4*, or *Equine influenza virus.*

In the methods of the present invention, in particular in step (c) thereof, the temperature can be fixed. That is, the one or more enzyme(s), DNA primers and the sample to be analyzed are incubated in the same tube at a constant temperature. The temperature is preferably between 50 and 75°C. However, the temperature may also be lower, for example between 30 and 75°C. In an alternative embodiment, a touchdown temperature step is used. That is, the temperature is lowered during the course of the analysis, for example starting at a temperature of 70°C that is subsequently lowered to 50°C.

In the methods of the present invention, the one or more enzyme(s), DNA primers and the sample to be analyzed are incubated in the same tube for a time between 1 and 120 minutes, preferably between 1 and 60, 1 and 45, 1 and 30 or between 1 and 15 minutes. In a preferred embodiment, the sample is incubated for 3, 4, 5, 6, 7, 8, 9, 10, 11, 12, 13, 14, 15, 16, 17, 18, 19, 20, 21, 22, 23, 24, 25, 26, 27, 28, 29 or 30 minutes.

Within the present invention, it is in one embodiment preferred that the virus is an *Equine influenza* virus. In such an embodiment of the invention, the preferred primer sequences are shown below:
(a) FIP primer sequence: CAA GTC TCT GCG CGA TCT AGG TCG AAA CGT ACG TTC (SEQ ID NO: 1); and/or
(b) BIP primer sequence: GAA GAT GTC TTT GCA GGG TTG GTC TTG TCT TTA GCC (SEQ ID NO: 2); and/or
(c) LPF primer sequence: TTT GAG GGG GCC TGA TGG (SEQ ID NO: 3); and/or
(d) LPB primer sequence: ACC GAT CTT GAG GCA CTC (SEQ ID NO: 4); and/or
(e) B3 primer sequence: AAT CCC TTT AGT YAG AG (SEQ ID NO: 5).

The above primer sequences target a sequence of *Equine influenza* virus RNA. In particular, the above primer target the following sequence
#JN850794.1_Influenza_A_virus_(A/equine/Newmarket/11/2003(H3N8)_segment_7_matrix _protein_2_(M2)_and_matrix_protein_1_(M1)_genes_complete_cds

ATGAGTCTTCTGACCGAGGTCGAAACGTACGTTCTCTCTATCGTACCATCAGGCC
CCCTCAAAGCCGAGATCGCGCAGAGACTTGAAGATGTCTTTGCAGGGAAGAACA
CCGATCTTGAGGCACTCATGGAATGGCTAAAGACAAGACCAATCCTGTCACCTTT
GACTAAAGGGATTTTAGGATTTGTATTCACGCTCACCGTGCCCAGTGAGCAAGGA

CTGCAGCGTAGACGCTTTGTCCAAAATGCCCTTAGTGGAAACGGAGATCCAAACA
ACATGGACAGAGCAGTAAAACTGTACAGGAAGCTTAAAAGAGAAATAACATTCC
ATGGGGCAAAAGAGGTGGCACTAAGCTATTCCACTGGTGCACTAGCCAGCTGCAT
GGGACTCATATACAACAGAATGGGAACTGTGACAACCGAAGTGGCATTTGGCCT
GGTATGCGCCACATGTGAACAGATTGCTGATTCCCAGCATCGATCTCACAGGCAG
ATGGTGACAACAACCAACCCATTAATCAGACATGAAAACAGAATGGTATTAGCC
AGTACCACGGCTAAAGCCATGGAACAGATGGCAGGATCAAGTGAGCAGGCAGCA
GAGGCCATGGAGGTTGCTAGTAAGGCTAGGCAGATGGTACAGGCAATGAGAACC
ATTGGGACCCACCCTAGCTCCAGTGCCGGTTTGAAAGATGATCTCCTTGAAAATT
TACAGGCCTACCAGAAACGGATGGGAGTGCAAATGCAGCGATTCAAGTGATCCT
CTCGTTATTGCAGCAAGTATCATTGGGATCTTGCACTTGATATTGTGGATTCTTGA
TCGTCTTTTCTTCAAATTCATTTATCGTCGCCTTAAATACGGGTTGAAAAGAGGGC
CTTCTACGGAAGGAGTACCTGAGTCTATGAGGGAAGAATATCGGCAGGAACAGC
AGAATGCTGTGGATGTTGACGATGGTCATTTTGTCAACATAGAGCTGGAGTAA
(SEQ ID NO : 13)

Within the present invention, it is in one embodiment preferred that the virus is an *Equine herpes* virus Type 1. In such an embodiment of the invention, the preferred primer sequences are shown below:
(a) FIP primer sequence: GTC GTA RAA CCT GAG AGC GGC CTG CTA GAC TAC AGC (SEQ ID NO: 7); and/or
(b) BIP primer sequence: CGA CAG CGT GGT CAA CGT TGA AAA AGC TGG CGA TCC (SEQ ID NO: 8); and/or
(c) LPF primer sequence: GAG CTG GTT GCG GCG CTG (SEQ ID NO: 9); and/or
(d) LPB primer sequence: ATA CCG CAG TGA TTA TGC (SEQ ID NO: 10); and/or
(e) B3 primer sequence: TCC CCC ACT TTA CCC AG (SEQ ID NO: 11).

The above primer sequences target a sequence of *Equine herpes* virus Type 1. In particular, the above primer target the following sequence:
#JN705797.1_Equid_herpesvirus_1_strain_TR04_glycoprotein_B_(glyB)_gene_partial_cds

GACACCACGCGAAGACGCAGGTCTCTCCTGTCAGTACCAGAACCTCAGCCAACCC
AAGATGGTGTGCATAGAGAACAAATTCTACATCGCTTGCACAAACGAGCAGTGG
AGGCAACGGCAGGTACCGATTCTTCCAACGTCACCGCCAAACAGCTGGAGCTCAT
CAAAACCACGTCGTCTATCGAGTTTGCCATGCTACAGTTTGCATACGATCACATC

CAATCCCACGTCAATGAAATGCTAAGTAGAATAGCAACTGCGTGGTGTACCCTCC
AAAACAAAGAGCGGACCCTATGGAACGAAATGGTGAAGATTAACCCGAGCGCCA
TAGTCTCCGCAACCCTTGACGAGCGAGTTGCAGCGAGGGTCCTGGGGGACGTGAT
AGCTATAACGCACTGCGCCAAAATAGAGGGCAACGTGTACTTGCAAAACTCCAT
GCGCTCGATGGACAGTAACACGTGCTACTCCCGCCCCCCCGTAACATTTACAATT
ACTAAGAATGCAAACAACAGAGGGTCGATAGAAGGCCAGCTGGGAGAGGAGAA
CGAGATTTTCACGGAGCGCAAGCTGATCGAGCCGTGCGCCCTCAATCAGAAGCGC
TACTTTAAGTTTGGCAAAGAGTACGTTTACTACGAGAACTACACGTTCGTCCGCA
AAGTGCCCCCCACGGAAATCGAGGTTATCAGCACGTACGTTGAACTAAACTTGAC
CCTTTTGGAAGACCGCGAGTTTCTGCCCCTGGAGGTGTACACGCGGGCTGAGCTG
GAGGACACCGGCCTGCTAGACTACAGCGAAATACAGCGCCGCAACCAGCTCCAC
GCTCTCAGGTTTTATGACATCGACAGCGTGGTCAACGTGGACAATACCGCAGTGA
TTATGCAGGGGATCGCCAGCTTTTTCAAGGGCCTGGGTAAAGTGGGGGAGGCCGT
GGGAACGCTCGTTCTCGGCGCCGCCGGCGCTGTTGTTTCAACCGT (SEQ ID NO:
14)

However, the skilled person is well-aware how to design alternative or further primer sequences depending on the target sequence to be detected in the sample.

The methods of the present invention comprise a step of determining whether a double-stranded elongated DNA sequence is present in the sample, in particular wherein presence of the double-stranded elongated DNA sequence in the sample is indicative of the presence of the pre-determined nucleic acid sequence in the sample. The skilled person is well-aware of methods suitable to be used for determining presence of a double-stranded DNA sequence in a sample, in particular where the sequence to be detected is known. Thus, any method known to the skilled person for that purpose may be used within the present invention. However, it is preferred that the presence of the elongated double-stranded DNA is determined by using a nucleic acid molecule hybridisable to the elongated double-stranded DNA sequence, in particular wherein the nucleic acid molecule is labelled, using a molecule that intercalates in the elongated double-stranded DNA sequence or using turbidity measurement.

In a further embodiment, the present invention relates to a method of treating an animal infected by a pathogen, the method comprising administering to the animal an efficient amount of a therapeutic drug, wherein the subject has previously been determined to be infected by the pathogen using the method of the present invention.

In a preferred embodiment, the pathogen is a virus, a bacterium, a fungus or a parasite. In a further preferred embodiment, the therapeutic drug is an antiviral, antibiotic, antifungal or antiparasitic drug, respectively. The skilled person is aware how to treat an infection with a pathogen once the pathogen has been specified using the methods of the present invention.

In a further embodiment, the invention relates to a kit, in particular a kit for use in detecting a nucleic acid molecule in a sample, in particular detecting infection of an animal with a pathogen. The kit comprises one or more, preferably all five or six primers for detecting a pre-determined sequence of the pathogen. The kit may also comprise more than one primer system, in particular two or more primer systems targeting different sequences of the same pathogen or different pathogens. As such, the methods of the invention and the kit of the invention can be used to detect more than one different pathogen in a sample by using more than one primer system. In this regard, it was surprisingly found that the reduced number of primers leads to an improved usability of more than one primer systems due to reduced primer interference.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the figures and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

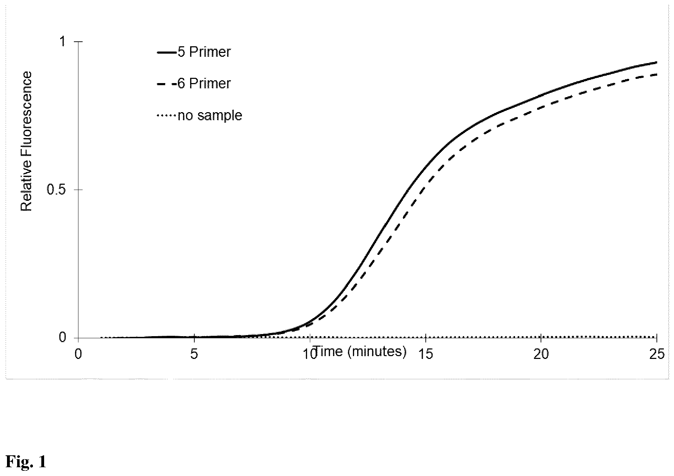

Figure 1 shows a comparison of the five and six primer systems on an *Equine influenza virus.*

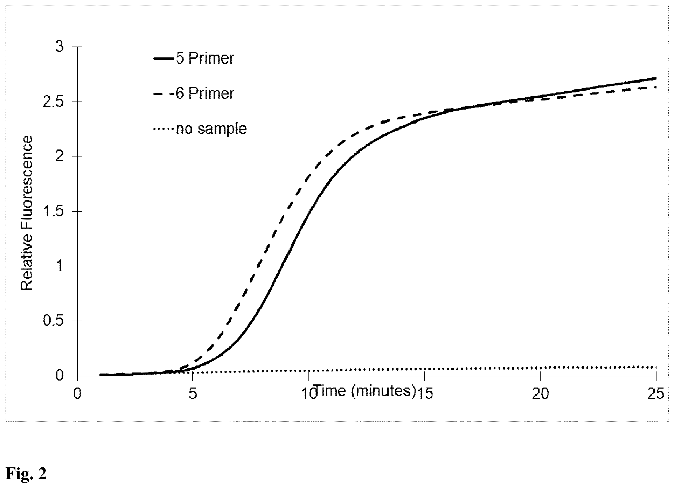

Figure 2 shows a comparison of the five and six primer systems on *Equine herpes virus 1.*

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

### Examples

The following are examples of methods and compositions of the invention. It is understood that various other embodiments may be practiced, given the general description provided above.

### The novel 5 primer system without F3 amplifies Equine influenza virus RNA as efficient as the 6 primer system with F3

**Table 1 - Primers**

| | | | |
|---|---|---|---|
| FIP | CAA GTC TCT GCG CGA TCT AGG TCG AAA CGT ACG TTC (SEQ ID NO: 1) | LPF | TTT GAG GGG GCC TGA TGG (SEQ ID NO: 3) |
| BIP | GAA GAT GTC TTT GCA GGG TTG GTC TTG TCT TTA GCC (SEQ ID NO: 2) | LPB | ACC GAT CTT GAG GCA CTC (SEQ ID NO: 4) |
| B3 | AAT CCC TTT AGT YAG AG (SEQ | F3 | AGT CTT CTG ACC GAG GTC |
| | ID NO: 5) | | (SEQ ID NO: 6) |

**Table 2 - Primer mix: novel 5 primer system**

| | **Final concentration.** |
|---|---|
| FIP | 1.6 µM |
| BIP | 1.6 µM |
| LPF | 0.8 µM |
| LPB | 0.8 µM |
| B3 | 0.4 µM |

**Table 3 - Primer mix: LAMP 6 primer system**

| | **Final concentration** |
|---|---|
| FIP | 1.6 µM |
| BIP | 1.6 µM |
| LPF | 0.8 µM |
| LPB | 0.8 µM |
| B3 | 0.2 µM |
| F3 | 0.2 µM |

**Table 4 - Primer/Enzyme mix (PEM)**

| | **Vol/rx** |
|---|---|
| **Isothermal master mix** | **15.0 µl** |
| **Primer mix** | **2.0 µl** |
| | **17.0 µl** |

| | |
|---|---|
| Add 17.0 µl PEM per reaction | |

### Template addition

Add 8.0 µl of template RNA
Add 8.0 µl RNase-free H2O as negative assay control

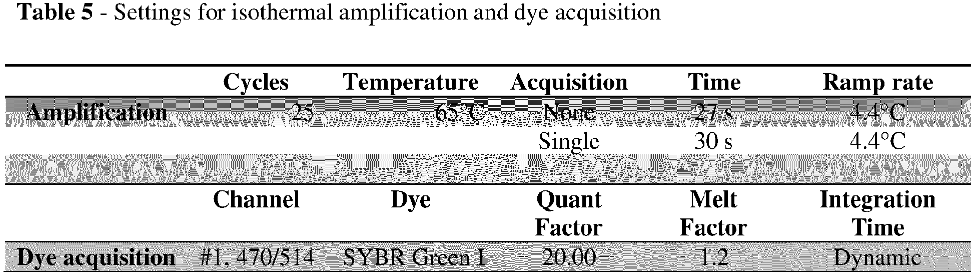

### The novel 5 primer system without F3 amplifies Equine herpes virus type 1 DNA as efficient as 6 primer system with F3

**Table 6 - Primers**

| | | | |
|---|---|---|---|
| FIP | GTC GTA RAA CCT GAG AGC GGC CTG CTA GAC TAC AGC (SEQ ID NO: 7) | LPF | GAG CTG GTT GCG GCG CTG (SEQ ID NO: 9) |
| BIP | CGA CAG CGT GGT CAA CGT TGA AAA AGC TGG CGA TCC (SEQ ID NO: 8) | LPB | ATA CCG CAG TGA TTA TGC (SEQ ID NO: 10) |
| B3 | TCC CCC ACT TTA CCC AG (SEQ ID NO: 11) | F3 | CTG AGC TGG AGG ACA C (SEQ ID NO: 12) |

**Table 7 - Primer mix: novel 5 primer system**

| | **Final concentration.** |
|---|---|
| FIP | 1.6 µM |
| BIP | 1.6 µM |
| LPF | 0.8 µM |
| LPB | 0.8 µM |
| B3 | 0.4 µM |

**Table 8 - Primer mix: LAMP 6 primer system**

| | **Final concentration** |
|---|---|
| FIP | 1.6 µM |
| BIP | 1.6 µM |
| LPF | 0.8 µM |
| LPB | 0.8 µM |
| B3 | 0.2 µM |
| F3 | 0.2 µM |

**Table 9 - Primer/Enzyme mix (PEM)**

| | **Vol/rx** |
|---|---|
| **Isothermal master mix** | **15.0 µl** |
| **Primer mix** | **2.0 µl** |
| | **17.0 µl** |

| | |
|---|---|
| Add 17.0 µl PEM per reaction | |

### Template addition

Add 8.0 µl template DNA
Add 8.0 µl RNase-free H2O as negative assay control

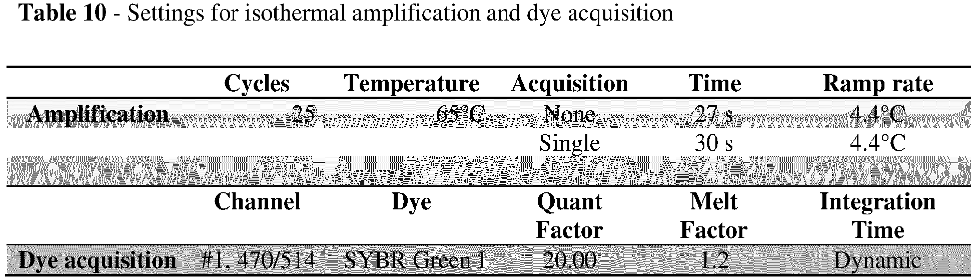

## Claims

1. A method for determining presence of a pre-determined nucleic acid sequence in a sample, the method comprising the steps of:
(a) adding one or more enzyme(s) providing activities of RNA- and/or DNA-dependent DNA polymerase activity and strand-displacement activity to the sample to be analysed for the presence of the pre-determined nucleic acid sequence;
(b) adding at least five DNA primers to the sample to be analysed for the presence of the pre-determined nucleic acid sequence, wherein at least one DNA primer comprises a sequence hybridisable to the nucleic acid sequence and at least one DNA primer comprises a sequence hybridisable to the DNA sequence reverse-complementary to the nucleic acid sequence;
(c) incubating the sample resulting from steps (a) and (b) at a fixed temperature;
(d) determining whether an elongated DNA sequence is present in the sample, wherein presence of the elongated DNA sequence in the sample is indicative of the presence of the pre-determined nucleic acid sequence in the sample
wherein the sample is obtained from an animal subject.

2. The method of claim 1, wherein four of the at least five primers are forward inner primer (FIP), backward inner primer (BIP), loop primer forward (LPF) and loop primer backwards (LPB), respectively.

3. The method of claim 1 and 2, wherein the fifth primer is a B3 primer.

4. The method of any one of claims 1 to 3, wherein no F3 primer is used.

5. The method of any one of claims 1 to 4, wherein the pre-determined nucleic acid sequence is an RNA or DNA sequence.

6. The method of any one of claims 1 to 5, wherein the pre-determined RNA or DNA sequence is comprised in a pathogen.

7. The method of claim 6, wherein the pathogen is a virus, a bacterium, a fungus or a parasite.

8. The method of any one of claims 1 to 7, wherein the fixed temperature is between 50 and 75°C.

9. The method of any one of claims 1 to 8, wherein the sample in step (c) is incubated for 1 to 120 minutes.

10. The method of any one of claims 1 to 9, wherein presence of the double-stranded elongated DNA sequence in the sample is determined by using a nucleic acid molecule hybridisable to the double-stranded elongated DNA sequence, in particular wherein the nucleic acid molecule is labelled, using a molecule that intercalates in the double-stranded elongated DNA sequence or using turbidity measurement.

11. A method of treating a non-subject infected by a pathogen, the method comprising administering to the subject an efficient amount of a therapeutic drug, wherein the subject has previously been determined to be infected by the pathogen using the method of any one of claims 1 to 10.

12. The method of claim 11 wherein the animal is a horse.

13. The method of claim 11 or 12, wherein the pathogen is a virus, a bacterium, a fungus or a parasite.

14. The method of any one of claims 11 or 13, wherein the therapeutic drug is an anti-viral, antibiotic, antifungal or antiparasitic drug, respectively.

15. The method of any one of claims 11 to 14, wherein the pathogen is *Streptococcus equi equi, Equine herpes virus,* in particular *Equine herpes virus 1* or *4,* or *Equine influenza virus.*


SEQUENCE LISTING

<110> LiVET AG

<120> Method for determining presence of a pre-determined nucleic acid
sequence in animal samples

<130> AD2231 EP BS

<160> 14

<170> BiSSAP 1.3.6

<210> 1
<211> 36
<212> DNA
<213> Artificial Sequence

<220>
<223> FIP primer Equine influenza virus

<400> 1
caagtctctg cgcgatctag gtcgaaacgt acgttc                                36

<210> 2
<211> 36
<212> DNA
<213> Artificial Sequence

<220>
<223> BIP primer Equine influenza virus

<400> 2
gaagatgtct ttgcagggtt ggtcttgtct ttagcc                                36

<210> 3
<211> 18
<212> DNA
<213> Artificial Sequence

<220>
<223> LPF primer Equine influenza virus

<400> 3
tttgaggggg cctgatgg                                                    18

<210> 4
<211> 18
<212> DNA
<213> Artificial Sequence

<220>
<223> LPB primer Equine influenza virus

<400> 4
accgatcttg aggcactc                                                    18

<210> 5
<211> 17
<212> DNA
<213> Artificial Sequence

<220>
<223> B3 primer Equine influenza virus

<400> 5
aatcccttta gtyagag                                                      17

<210> 6
<211> 18
<212> DNA
<213> Artificial Sequence

<220>
<223> F3 primer Equine influenza virus

<400> 6
agtcttctga ccgaggtc                                                     18

<210> 7
<211> 36
<212> DNA
<213> Artificial Sequence

<220>
<223> FIP primer Equine herpes virus type 1

<400> 7
gtcgtaraac ctgagagcgg cctgctagac tacagc                                 36

<210> 8
<211> 36
<212> DNA
<213> Artificial Sequence

<220>
<223> BIP primer Equine herpes virus type 1

<400> 8
cgacagcgtg gtcaacgttg aaaaagctgg cgatcc                                 36

<210> 9
<211> 18
<212> DNA
<213> Artificial Sequence

<220>
<223> LPF primer Equine herpes virus type 1

<400> 9
gagctggttg cggcgctg                                                    18

<210> 10
<211> 18
<212> DNA
<213> Artificial Sequence

<220>
<223> LPB primer Equine herpes virus type 1

<400> 10
ataccgcagt gattatgc                                                    18

<210> 11
<211> 17
<212> DNA
<213> Artificial Sequence

<220>
<223> B3 primer Equine herpes virus type 1

<400> 11
tcccccactt tacccag                                                     17

<210> 12
<211> 16
<212> DNA
<213> Artificial Sequence

<220>
<223> F3 primer Equine herpes virus type 1

<400> 12
ctgagctgga ggacac                                                      16

<210> 13
<211> 982
<212> DNA
<213> Artificial Sequence

<220>
<223> Influenza A virus (A/equine/Newmarket/11/2003(H3N8)) segment 7
      matrix protein 2 (M2) and matrix protein 1 (M1) genes complete
      cds

<400> 13
atgagtcttc tgaccgaggt cgaaacgtac gttctctcta tcgtaccatc aggccccctc      60

aaagccgaga tcgcgcagag acttgaagat gtctttgcag ggaagaacac cgatcttgag     120

gcactcatgg aatggctaaa gacaagacca atcctgtcac ctttgactaa agggatttta     180

ggatttgtat tcacgctcac cgtgcccagt gagcaaggac tgcagcgtag acgctttgtc     240

caaaatgccc ttagtggaaa cggagatcca aacaacatgg acagagcagt aaaactgtac 300

aggaagctta aaagagaaat aacattccat ggggcaaaag aggtggcact aagctattcc 360

actggtgcac tagccagctg catgggactc atatacaaca gaatgggaac tgtgacaacc 420

gaagtggcat ttggcctggt atgcgccaca tgtgaacaga ttgctgattc ccagcatcga 480

tctcacaggc agatggtgac aacaaccaac ccattaatca gacatgaaaa cagaatggta 540

ttagccagta ccacggctaa agccatggaa cagatggcag gatcaagtga gcaggcagca 600

gaggccatgg aggttgctag taaggctagg cagatggtac aggcaatgag aaccattggg 660

acccacccta gctccagtgc cggtttgaaa gatgatctcc ttgaaaattt acaggcctac 720

cagaaacgga tgggagtgca aatgcagcga ttcaagtgat cctctcgtta ttgcagcaag 780

tatcattggg atcttgcact tgatattgtg gattcttgat cgtcttttct tcaaattcat 840

ttatcgtcgc cttaaatacg ggttgaaaag agggccttct acggaaggag tacctgagtc 900

tatgagggaa gaatatcggc aggaacagca gaatgctgtg gatgttgacg atggtcattt 960

tgtcaacata gagctggagt aa 982

<210> 14
<211> 974
<212> DNA
<213> Artificial Sequence

<220>
<223> Equid herpesvirus 1 strain TR04 glycoprotein B (glyB)

<400> 14
gacaccacgc gaagacgcag gtctctcctg tcagtaccag aacctcagcc aacccaagat 60

ggtgtgcata gagaacaaat tctacatcgc ttgcacaaac gagcagtgga ggcaacggca 120

ggtaccgatt cttccaacgt caccgccaaa cagctggagc tcatcaaaac cacgtcgtct 180

atcgagtttg ccatgctaca gtttgcatac gatcacatcc aatcccacgt caatgaaatg 240

ctaagtagaa tagcaactgc gtggtgtacc ctccaaaaca aagagcggac cctatggaac 300

gaaatggtga agattaaccc gagcgccata gtctccgcaa cccttgacga gcgagttgca 360

gcgagggtcc tgggggacgt gatagctata acgcactgcg ccaaaataga gggcaacgtg 420

tacttgcaaa actccatgcg ctcgatggac agtaacacgt gctactcccg cccccccgta 480

acatttacaa ttactaagaa tgcaaacaac agagggtcga tagaaggcca gctgggagag 540

gagaacgaga ttttcacgga gcgcaagctg atcgagccgt gcgccctcaa tcagaagcgc 600

tactttaagt ttggcaaaga gtacgtttac tacgagaact acacgttcgt ccgcaaagtg 660

ccccccacgg aaatcgaggt tatcagcacg tacgttgaac taaacttgac ccttttggaa 720

gaccgcgagt ttctgcccct ggaggtgtac acgcgggctg agctggagga caccggcctg 780

ctagactaca gcgaaataca gcgccgcaac cagctccacg ctctcaggtt ttatgacatc 840

gacagcgtgg tcaacgtgga caataccgca gtgattatgc aggggatcgc cagctttttc 900

aagggcctgg gtaaagtggg ggaggccgtg ggaacgctcg ttctcggcgc cgccggcgct 960

gttgtttcaa ccgt 974